# EUROPEAN PATENT APPLICATION

(11) **EP 4 650 341 A1**
(43) Date of publication of application: **19.11.2025**
(21) Application number: 24910376.3
(22) Date of filing: 14.11.2024
(51) Int. Cl.: C07C 213/00, B01J 20/22, C02F 1/28

(54) **ABSORBENT FOR CAPTURING ACID GASES, AND PREPARATION METHOD THEREFOR AND USE THEREOF**

(30) Priority: 26.12.2023 CN 202311811038
(71) Applicant: Zhang, Zhaofu, Beijing 100091 (CN)
(72) Inventor: Zhang, Zhaofu, Beijing 100091 (CN)
(74) Representative: Bryn Aarflot AS
(86) International application number: PCT/CN2024/132028
(87) International publication number: WO 2025/139440

(57) **Abstract**

The present application relates to the field of the absorption and regeneration of acid gases, and specifically relates to an absorbent for capturing acid gases, and a preparation method therefor and the use thereof. A compound for capturing acid gases provided by the present application has a structure as represented by formula (I), wherein R₁ is an alkyl group having five or more carbon atoms, M is at least one of a proton-bound organic amine group and a proton-bound heterocyclic group containing a nitrogen atom, and n is an integer from 1 to 4. The compound for capturing acid gases provided by the present application has a relatively weak alkalinity and a relatively small acting force towards acid gases; moreover, the generation of a solid can be avoided during the absorption of acid gases with the compound, and therefore difficulties in the regeneration of the acid gases are reduced, and the regeneration process is relatively easy. The compound provided by the present application has a higher absorption rate for acid gases in the case of a relatively high water content.

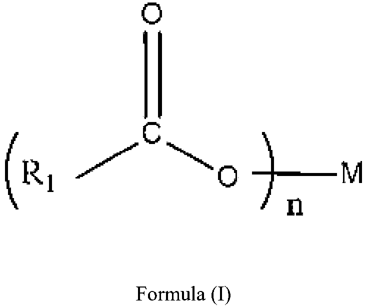

## Description

### CROSS REFERENCE TO RELATED APPLICATIONS

The present application claims priority to Chinese Patent Application No. 2023118110382 filed on December 26, 2023 with China National Intellectual Property Administration entitled "ABSORBENT FOR CAPTURING ACID GASES, AND PREPARATION METHOD THEREFOR AND USE THEREOF", the entire contents of which are incorporated herein by reference.

### TECHNICAL FIELD

The present application relates to the field of acid gas absorption and regeneration, specifically to an absorbent for capturing acid gases and its preparation method and use.

### BACKGROUND

The large-scale emission of acid gases such as sulfur dioxide and carbon dioxide not only affects the ecological environment and human health but also results in resource waste. Therefore, the absorption and regeneration of acid gases have become an important approach for resource recovery and utilization. Acid gas capture can be achieved through techniques such as chemical absorption, pressure swing adsorption, and membrane separation, among which chemical absorption is the most widely used method, primarily employing alkanolamine absorbents for acid gas absorption. However, alkanolamines react strongly with carbon dioxide, resulting in high reaction heat and regeneration temperatures between 105-120°C, leading to high energy consumption for regeneration and increased energy loss during acid gas regeneration. Currently, researchers have explored the use of carboxylate salts and carboxylate-based ionic liquids for acid gas absorption, but these materials have high viscosity. Although increasing water concentration can reduce the viscosity of the system, the increase of water content also leads to a decrease in acid gas absorption rate.

Existing technology has disclosed the application of carboxylate salt based compounds as absorbents for capturing carbon dioxide, where quaternary ammonium or phosphonium ions serve as the cations of the carboxylate salts to improve CO₂ absorption rate. However, these materials tend to form solids during the absorption process of the acid gases such as CO₂, making separation process inconvenient.

### SUMMARY OF THE INVENTION

Therefore, the present application aims to address the issue that it tends to produce solid during the absorption of acid gases by carboxylate salts in existing technology, thereby providing an absorbent for capturing acid gases as well as its preparation method and application to improve the absorption rate of acid gases and reduce the difficulty of acid gas regeneration.

On one hand, the present application provides a compound for capturing acid gases, wherein the compound has a structure shown in Formula (I), 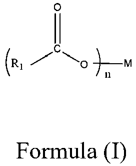 wherein, R₁ is an alkyl group with the number of carbon atoms greater than or equal to 5, M is at least one of protonated organic amine groups and protonated nitrogen-containing heterocyclic groups, and n is an integer from 1 to 4.

In one embodiment, R₁ is a branched alkyl group.

Optionally, the number of carbon atoms of R₁ is 6-20.

In one embodiment, the organic amine is at least one of fatty amine and alcohol amine.

Optionally, the organic amine is at least one of monoamine, diamine, and polyamine.

In one embodiment, the nitrogen-containing heterocycle is at least one of 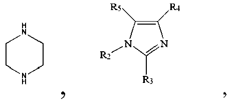 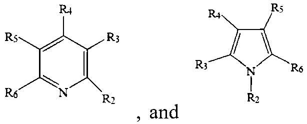 , where R₂, R₃, R₄, R₅, R₆ are any one of alkyl, substituted alkyl, or hydrogen atom.

In one embodiment, the compound for capturing acid gases has any one of the following structures:

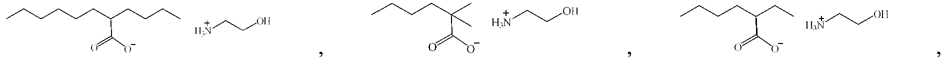

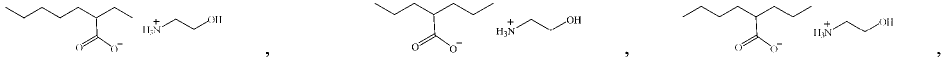

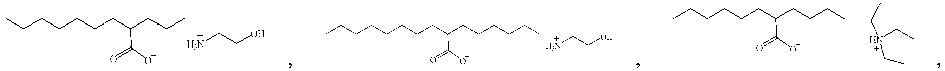

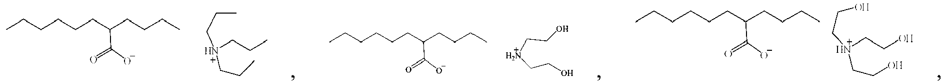

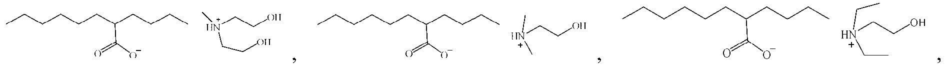

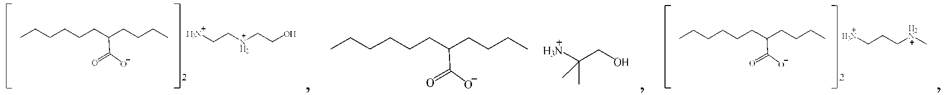

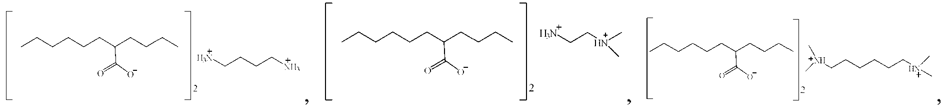

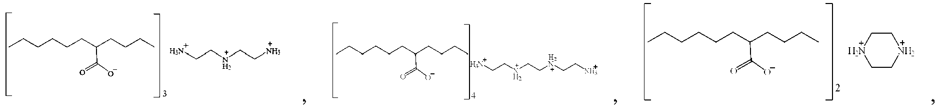

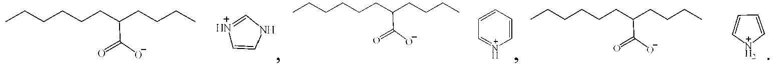

The present application also provides an absorbent for capturing acid gases, which includes the compound for capturing acid gases as described above.

On the other hand, the present application provides a method for preparing the absorbent for capturing acid gases, which includes the following steps: mixing a carboxylic acid and an organic weak base to obtain the absorbent, where the carboxylic acid has a molecular formula of R₁COOH, and R₁ is an alkyl group with the number of carbon atoms greater than or equal to 5.

In one embodiment, the absorbent also includes water.

In one embodiment, the carboxylic acid is fatty acid. Optionally, the carboxylic acid is at least one of 2,2-dimethylhexanoic acid, 2-ethylhexanoic acid, 2-ethylheptanoic acid, 2-propylvaleric acid, 2-propylhexanoic acid, 2-propylnonanoic acid, 2-butyl-octanoic acid, and 2-hexyldecanoic acid.

In one embodiment, the organic weak base is at least one of an organic amine compound and a nitrogen-containing heterocyclic compound.

Optionally, the organic amine compound is at least one of triethylamine, tripropylamine, monoethanolamine, diethanolamine, triethanolamine, N-methyldiethanolamine, N,N-dimethylethanolamine, N,N-diethylethanolamine, hydroxyethyl ethylenediamine, 2-amino-2-methyl-1-propanol, N-methyl-1,3-propanediamine, 1,4-butanediamine, N,N,N',N'-tetramethylhexanediamine, diethylenetriamine, and triethylenetetramine.

Optionally, the nitrogen-containing heterocyclic compound is at least one of a pyridine ring-containing compound, an imidazole ring-containing compound, a pyrrole ring-containing compound, and piperazine.

In one embodiment, a molar ratio of the carboxylic acid, the organic weak base, and water is 1:(0.25-1):(0-20).

The present application also provided use of the compounds for capturing acid gases, the absorbents for capturing acid gases, or the absorbents prepared by the method for preparing absorbents for capturing acid gases in absorbing and regenerating acid gases, wherein the acid gas is at least one of carbon dioxide, sulfur dioxide, or hydrogen sulfide.

Absorbing acid gases includes steps of contacting the absorbent with the acid gases to be absorbed at an acid gas partial pressure of 0.003 MPa - 1.0 MPa and a temperature of 0-50°C and performing absorption.

Regenerating acid gases includes a step of desorbing the absorbent after absorbing the acid gases under stirring for 0.1-2 hours at 80-100 °C, or, regenerating acid gases comprises a step of desorbing the absorbent after absorbing the acid gases under stirring for 0.1-1 hours at 10-80 °C under vacuum conditions.

The technical solutions provided by the present application have the following advantages:
1. The compound for capturing acid gases provided by the present application has a structure represented by Formula (I), where R₁ is an alkyl group with a carbon atom number greater than or equal to 5, M is at least one of protonated organic amine groups and protonated nitrogen-containing heterocyclic group, and n is an integer from 1 to 4. On the one hand, the compound for capturing acid gases provided by the present application has weaker basicity and thus has a smaller interaction force with acid gases, and this compound can avoid the formation of solids during acid gas absorption, and reduce the difficulty of regenerating acid gases, thereby making the regeneration process relatively easier. On the other hand, under higher water content, the compound provided by the present application has a higher absorption rate for acid gases.
2. In the compound for capturing acid gases provided by the present application, R₁ is a branched alkyl group. As the carbon chain increases, it can improve the compound's absorption rate for acid gases. At the same time, a branched alkyl group has a weaker interaction force with water, therefore, after absorption of carbon dioxide, the formed organic phase and water phase are more stable.
3. The absorbent for capturing acid gases provided by the present application includes the aforementioned compound for capturing acid gases. The absorbent provided by the present application is a weak acid weak base salt. Under higher water content, the absorbent can absorb a greater amount of acid gases, and does not generate solids during absorption of acid gases, thereby not increasing the difficulty of regenerating the acid gases, and improving the regeneration rate of acid gases.
4. The method for preparing the absorbent for capturing acid gases provided by the present application involves mixing a carboxylic acid, an organic weak bases, and water to obtain the absorbent, where the carboxylic acid has a molecular formula of R₁COOH, and R₁ is an alkyl group with a number of carbon atoms greater than or equal to 5. The present application only requires a mixing step of the carboxylic acid and organic weak base to create the weak acid weak base salt, making the synthesis method simple.
5. The aforementioned compound, absorbent, and absorbent prepared by the aforementioned method provided by the present application can absorb and regenerate acid gases. The regeneration steps include heating the absorbent after absorbing acid gases to 100°C or stirring the absorbent after absorbing acidic gases under vacuum conditions for a certain period of time to complete the regeneration. The aforementioned compound, absorbent, and absorbent prepared by the aforementioned method provided by the present application can desorb the absorbed acid gases at 100°C or by using negative pressure at room temperature, significantly reducing the energy consumption for the regeneration of acid gases.

### ILLUSTRATION OF THE DRAWINGS

To more clearly illustrate specific embodiments of the present application or the technical solutions of existing technologies, a brief introduction to the drawings needed in the description of specific embodiments or existing technologies will be provided below. It is obvious that the drawings described below are some implementations of the present application. In the absence of creative labor, ordinary technicians in this field can obtain other drawings based on these drawings.

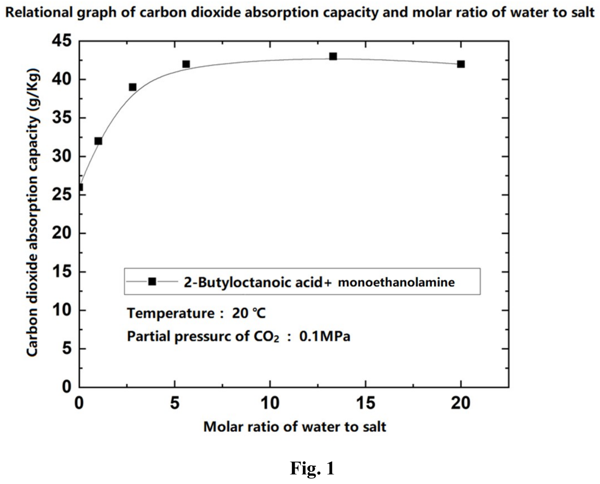

Figure 1 is a schematic diagram of the absorption quantity of carbon dioxide absorbed by the absorbents made in Examples 1, 28-32 of the present application.

### DETAILED DESCRIPTION

The following examples are provided to better understand the present application and are not limited to the best implementation mode. They do not constitute a limitation on the content and scope of protection of the present application. Any product that is identical or similar to the present application, whether derived from the insights of the present application or by combining the features of the present application with the features of other existing technologies, falls within the scope of protection of the present application.

If specific experimental steps or conditions are not specified in the examples, the operations or conditions can be carried out according to the conventional experimental steps described in the literature within the field. If the manufacturer of the reagents or instruments used is not specified, they are all conventional reagent and products that can be purchased commercially.

During the research and development process, it was found that the water-to-salt ratio of existing acid gas carboxylate salt based absorbents significantly affects the absorption rate of acid gases. The higher the water-to-salt ratio, the lower the amount of acid gases absorbed by the absorbent. Moreover, with the increase in the absorption quantity of acid gases, solids can form in the absorption system. The formed solids not only cause inconvenience in subsequent operations, such as blocking pipes and causing equipment damage, but also add additional procedures for removing the solids, thereby making it more difficult to regenerate the acid gases and reducing their regeneration rate.

Therefore, the inventor developed a method to generate weak acid weak base salts by reacting carboxylic acids with organic weak bases. For example, the weak acid weak base salt obtained by the reaction of 2-butyl octanoic acid with monoethanolamine, as shown in Formula (II), can effectively address the above issues.

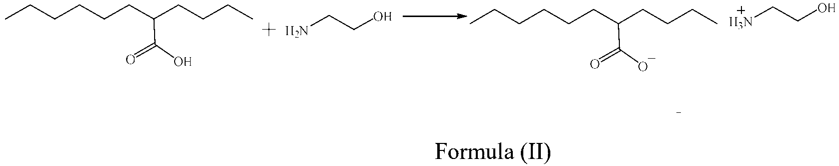

The carboxylate onium salt in Comparative Examples 5-9 of the present application were synthesized using mature technologies, with the synthesis method referenced from the literature: Suarez, P. A. Z.; Dullius, J. E. L.; Einloft, S.; DE Souza R. F. and Dupont, J., Polyhedron, 1996, 15, 1217-1219.

### Examples 1-27

Examples 1-27 provide methods for preparing absorbents for capturing acid gases, with the specific steps and parameters as follows:
According to the names and amounts of the carboxylic acid, organic weak base, and water listed in Table 1, various raw materials were mixed and stirred to obtain the absorbents.

**Table 1. Absorbent preparation information and structural formulae**

| Group No. | Carboxylic acid name /amount (g) | Base name / amount (g) | Water amount (g) | Absorbent structural formula |
|---|---|---|---|---|
| Example 1 | 2-butyl octanoic acid /20.0 | Monoethanolami ne (MEA) /6.1 | 23.9 | 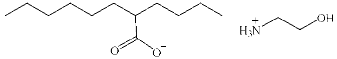 |
| Example 2 | 2,2-dimethylhex anoic acid /14.4 | MEA /6.1 | 23.9 | 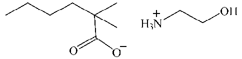 |
| Example 3 | 2-ethylhexanoic acid /14.4 | MEA/6.1 | 23.9 | 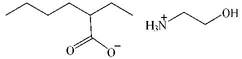 |
| Example 4 | 2-ethylheptanoic acid /15.8 | MEA/6.1 | 23.9 | 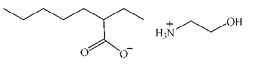 |
| Example 5 | 2-propylvaleric acid /14.4 | MEA/6.1 | 23.9 | 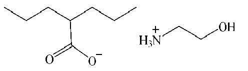 |
| Example 6 | 2-propylhexanoi c acid /15.8 | MEA/6.1 | 23.9 | 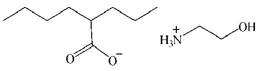 |
| Example 7 | 2-propylnonanoi c acid /20.0 | MEA/6.1 | 23.9 | 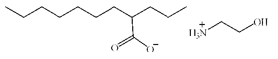 |
| Example 8 | 2-hexyldecanoic acid /25.6 | MEA/6.1 | 23.9 | 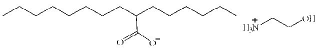 |
| Example 9 | 2-butyloctanoic acid /20.0 | Triethylamine /10.1 | 23.9 | 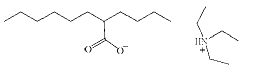 |
| Example 10 | 2-butyloctanoic acid /20.0 | Tripropylamine /14.3 | 23.9 | 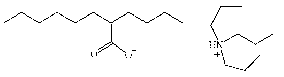 |
| Example 11 | 2-butyloctanoic acid /20.0 | Diethanolamine /10.5 | 23.9 | 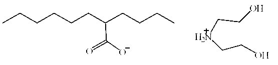 |
| Example 12 | 2-butyloctanoic acid /20.0 | Triethanolamine /14.9 | 23.9 | 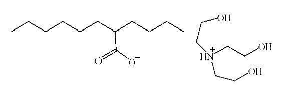 |
| Example 13 | 2-butyloctanoic acid /20.0 | N-methyldiethan olamine /11.9 | 23.9 | 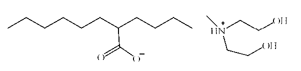 |
| Example 14 | 2-butyloctanoic acid /20.0 | N,N-dimethyleth anolamine /8.9 | 23.9 | 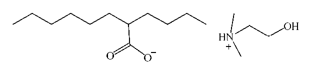 |
| Example 15 | 2-butyloctanoic acid /20.0 | N,N-diethylethanola mine /11.7 | 23.9 | 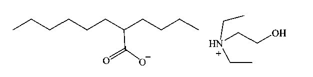 |
| Example 16 | 2-butyloctanoic acid /20.0 | Hydroxyethyl ethylenediamine /5.2 | 23.9 | 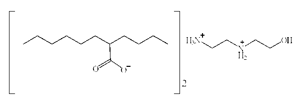 |
| Example 17 | 2-butyloctanoic acid /20.0 | 2-amino-2-meth yl-1-propanol /8.9 | 23.9 | 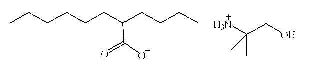 |
| Example 18 | 2-butyloctanoic acid /20.0 | N-methyl-1,3-pr opanediamine /4.4 | 23.9 | 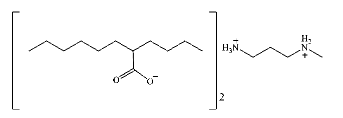 |
| Example 19 | 2-butyloctanoic acid /20.0 | 1,4-butanediami ne /4.4 | 23.9 | 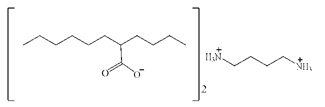 |
| Example 20 | 2-butyloctanoic acid /20.0 | N,N-dimethyl ethylenediamine /4.4 | 23.9 | 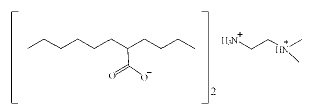 |
| Example 21 | 2-butyloctanoic acid /20.0 | N,N,N',N'-tetramethyl-1,6-hexanediamine /8.6 | 23.9 | 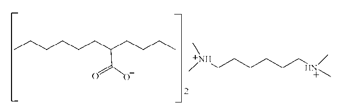 |
| Example 22 | 2-butyloctanoic acid /20.0 | Diethylene triamine /3.4 | 23.9 | 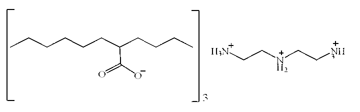 |
| Example 23 | 2-butyloctanoic acid /20.0 | Triethylene tetramine /3.7 | 23.9 | 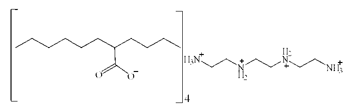 |
| Example 24 | 2-butyloctanoic acid /20.0 | Piperazine /4.5 | 23.9 | 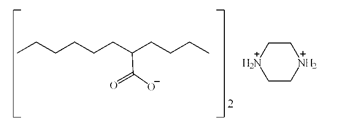 |
| Example 25 | 2-butyloctanoic acid/20.0 | Imidazole /6.8 | 23.9 | 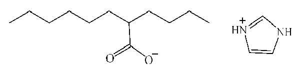 |
| Example 26 | 2-butyloctanoic acid/20.0 | Pyridine /7.9 | 23.9 | 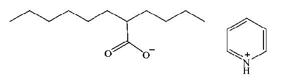 |
| Example 27 | 2-butyloctanoic acid/20.0 | Pyrrole /6.7 | 23.9 | 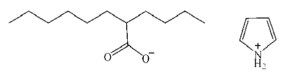 |
| Example 28 | 2-butyloctanoic acid/20.0 | MEA/6.1 | 0 | 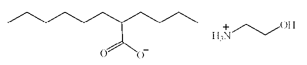 |
| Example 29 | 2-butyloctanoic acid/20.0 | MEA/6.1 | 1.8 | 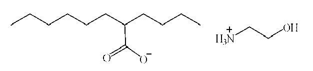 |
| Example 30 | 2-butyloctanoic acid/20.0 | MEA/6.1 | 5 | 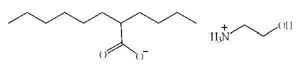 |
| Example 31 | 2-butyloctanoic acid/20.0 | MEA/6.1 | 10 | 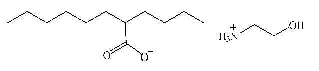 |
| Example 32 | 2-butyloctanoic acid/20.0 | MEA/6.1 | 36 | 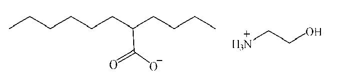 |
| Example 33 | 2-butyloctanoic acid/20.0 | Triethylenetetra mine /3.7 | 18.0 | 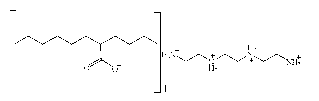 |
| Comparative Example 1 | 2-butyloctanoic acid/20.0 | | 23.9 | 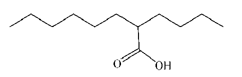 |
| Comparative Example 2 | MEA/6.1 | | 23.9 | 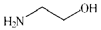 |
| Comparative Example 3 | Potassium Acetate /8.9 | | 23.9 | 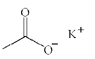 |
| Comparative Example 4 | Acetic Acid /6.0 | MEA/6.1 | 23.9 | 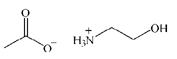 |
| Comparative Example 5 | Triethylbutylammonium Isobutyrate /24.5 | | 23.9 | 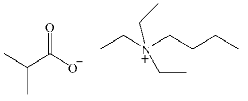 |
| Comparative Example 6 | Tributylhexylphosphonium Acetate /34.6 | | 23.9 | 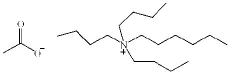 |

### Examples 28-32

Examples 28-32 provided preparation methods for absorbents for capturing acid gases, with the specific steps and parameters as follows:
2-butyloctanoic acid, monoethanolamine, and deionized water were mixed in molar ratios of 1:1:0, 1:1:1, 1:1:2.8, 1:1:5.6, and 1:1:20 and stirred uniformly to obtain the absorbents.

### Example 33

This example provided a method for preparing an absorbent for capturing acid gases, with the specific steps and parameters as follows:
2-butyloctanoic acid, triethylenetetramine, and deionized water were mixed in a molar ratio of 1:0.25:10 and stirred uniformly to obtain the absorbent.

### Comparative Example 1

This comparative example provided a method for preparing an absorbent for capturing acid gases, with the specific steps and parameters as follows:
0.1 mol of 2-butyloctanoic acid and 1.33 mol of water were directly mixed and stirred to obtain the absorbent.

### Comparative Example 2

This comparative example provided a method for preparing an absorbent for capturing acid gases, with the specific steps and parameters as follows:
0.1 mol of monoethanolamine (MEA) and 1.33 mol of water were directly mixed and stirred to obtain the absorbent.

### Comparative Example 3

This comparative example provided a method for preparing an absorbent for capturing acid gases, with the specific steps and parameters as follows:
0.1 mol of potassium acetate and 1.33 mol of water were mixed to obtain the absorbent.

### Comparative Example 4

This comparative example provided a method for preparing an absorbent for capturing acid gases, with the specific steps and parameters as follows:
0.1 mol of acetic acid, 0.1 mol of MEA, and 1.33 mol of water were mixed to obtain the absorbent.

### Comparative Example 5

This comparative example provided a method for preparing an absorbent for capturing acid gases, with the specific steps and parameters as follows:
0.1 mol of triethylbutylammonium isobutyrate and 1.33 mol of water were mixed to obtain the absorbent.

### Comparative Example 6

This comparative example provided a method for preparing an absorbent for capturing acid gases, with the specific steps and parameters as follows:
0.1 mol of tributylhexylphosphonium acetate and 1.33 mol of water were mixed to obtain the absorbent.

### Comparative Example 7

This comparative example provided a method for preparing an absorbent for capturing acid gases, with the specific steps and parameters as follows:
0.1 mol of tributylhexylphosphonium 2-ethylhexanoate and 10g of water were mixed to obtain the absorbent.

### Comparative Example 8

This comparative example provided a method for preparing an absorbent for capturing acid gases, with the specific steps and parameters as follows:
0.1 mol of trimethylbutylammonium 2-butyloctanoate and 10g of water were mixed to obtain the absorbent.

### Comparative Example 9

This comparative example provided a method for preparing an absorbent for capturing acid gases, with the specific steps and parameters as follows:
0.1 mol of potassium 2-butyloctanoate and 10g of water were mixed to obtain the absorbent.

### Application Example 1

The absorbents prepared by Examples 1-33 and Comparative Examples 1-6, each 50g, were injected into glass bottles, respectively. The bottles were connected to CO₂ balloons, and the absorbent solution was immersed in CO₂ at one atmospheric pressure at 20°C under stirring for 3 hours. The weight of the bottles is recorded before and after CO₂ absorption, and the amount of CO₂ absorbed is calculated from the weight gain of the system. The results for CO₂ absorption quantity absorbed by absorbents prepared by Examples 1-33 and Comparative Examples 1-6 were shown in Table 2.

**Table 2: Results of CO₂ Absorption quantity and Absorption Rate**

| Group No. | CO₂ absorption quantity (g) | CO₂ absorption rate (%) | Group No. | CO₂ absorption quantity (g) | CO₂ absorption rate (%) | Group No. | CO₂ absorption quantity (g) | CO₂ absorption rate (%) |
|---|---|---|---|---|---|---|---|---|
| Example 1 | 2.13 | 4.3 | Example 14 | 1.80 | 3.6 | Example 27 | 1.59 | 3.2 |
| Example 2 | 1.14 | 2.3 | Example 15 | 1.76 | 3.5 | Example 28 | 1.25 | 2.6 |
| Example 3 | 1.16 | 2.3 | Example 16 | 1.78 | 3.6 | Example 29 | 1.60 | 3.2 |
| Example 4 | 1.34 | 2.7 | Example 17 | 1.93 | 3.9 | Example 30 | 1.96 | 3.9 |
| Example 5 | 1.18 | 2.4 | Example 18 | 1.93 | 3.9 | Example 31 | 2.08 | 4.2 |
| Example 6 | 1.42 | 2.8 | Example 19 | 1.85 | 3.7 | Example 32 | 2.10 | 4.2 |
| Example 7 | 1.82 | 3.6 | Example 20 | 1.62 | 3.2 | Example 33 | 1.61 | 3.2 |
| Example 8 | 2.04 | 4.1 | Example 21 | 1.62 | 3.2 | Comparative Example 1 | 0.15 | 0.3 |
| Example 9 | 2.62 | 5.2 | Example 22 | 1.70 | 3.4 | Comparative Example 2 | 4.13 | 8.2 |
| Example 10 | 2.40 | 4.8 | Example 23 | 1.66 | 3.3 | Comparative Example 3 | 0.56 | 1.1 |
| Example 11 | 1.81 | 3.6 | Example 24 | 1.93 | 3.9 | Comparative Example 4 | 0.39 | 0.8 |
| Example 12 | 1.49 | 3.0 | Example 25 | 1.72 | 3.4 | Comparative Example 5 | 0.49 | 1.0 |
| Example 13 | 1.50 | 3.0 | Example 26 | 1.61 | 3.2 | Comparative Example 6 | 0.47 | 0.9 |

The CO₂ absorption rate is defined as the CO₂ absorption quantity divided by the mass of the absorbent.

According to Table 2, it can be seen that, the absorbents prepared by the present application by mixing carboxylic acid and organic weak base were effective in absorbing CO₂, with absorption rates ranging from 2.3% to 5.2%. Comparative Example 1 shows low CO₂ absorption quantity using only carboxylic acid, while Comparative Example 3 uses the carboxylate salts formed by using short-chain aliphatic alkyl carboxylic acids and strong bases, Comparative Example 4 uses absorbent prepared by short-chain aliphatic alkyl carboxylic acids and organic weak bases, Comparative Example 5 uses quaternary ammonium salts of short-chain aliphatic alkyl carboxylic acids, and Comparative Example 6 uses quaternary phosphonium salts of short-chain aliphatic alkyl carboxylic acids, respectively, Comparative Examples 3, 4, 5, and 6 showed CO₂ absorption rates of only 0.8% to 1.1%.

As shown in Figure 1, the molar ratios of water to salt in Example 28, Example 29, Example 30, Example 31, Example 1, and Example 32 were 0, 1:1, 2.8:1, 5.6:1, 13.3:1, and 20:1, respectively. It can be seen from Figure 1, increasing the molar ratio of water to salt in the absorbents can increase the CO₂ absorption quantity of the absorbents.

### Application Example 2

The glass bottles containing the absorbents from Example 1 and Comparative Example 2, after absorbing CO₂ as described in Application Example 1, were stirred at 100°C in an oil bath, while the glass bottle containing the absorbent from Example 14 after absorbing CO₂ was stirred at 80°C in an oil bath. The outlet of each of the glass bottles was connected to a spiral stainless steel tube submerged in ice water to trap moisture in the gas. After different regeneration times, the CO₂ desorption quantity was determined according to the total weight change of the glass bottles and the stainless steel tubes. The results were shown in Table 3.

**Table 3. Comparison of Thermal Regeneration Performance of Absorbents**

| Group No. | CO₂ absorption quantity /g | Regeneration oil bath temperature /°C | Regeneration time /h | CO₂ desorption quantity /g | Desorption rate /% |
|---|---|---|---|---|---|
| Example 1 | 2.13 | 100 | 1 | 2.02 | 95 |
| Example 1 | 2.13 | 100 | 0.1 | 0.81 | 38 |
| Example 14 | 1.80 | 80 | 2 | 1.45 | 80 |
| Comparative Example 2 | 4.13 | 100 | 1 | 0.32 | 8 |

It can be seen from Table 3, the absorbents obtained in the present application had good desorption effect for the absorbed carbon dioxide. At 80°C, the desorption rate can reach 80%, and at 100°C, the desorption rate can reach 95%. In Comparative Example 2, the organic weak base was used as the absorbent, although the absorption quantity of carbon dioxide was relatively large, the desorption rate of carbon dioxide by thermal regeneration was only 8%.

It can be seen from Table 2 and Table 3, in Comparative Example 1, where the carboxylic acid was used as the absorbent, there was only a small amount of physical absorption of carbon dioxide. In Comparative Example 2, where the organic weak base was used as the absorbent, the desorption rate of carbon dioxide under 100°C was extremely low. This suggested that, the absorption and regeneration effects for carbon dioxide by the weak acid weak base salt absorbents obtained in the present application, were achieved by the function of the product of the reaction between carboxylic acid and weak base, not due to the effects of either carboxylic acid or weak base individually.

Under normal temperature conditions, the glass bottle containing the absorbents from Example 1, after absorbing CO₂ as described in Application Example 1, was connected to a water pump, the glass bottle was vacuumed to maintain a negative pressure state, and the glass bottle was placed on a stirrer and stirred for 1 hour. There was a large amount of bubbling, and the system transitions from two phases to a homogeneous phase.

Under normal temperature conditions, the glass bottle containing the absorbents from Example 14, after absorbing CO₂ as described in Application Example 1, was connected to a water pump, the glass bottle was vacuumed to maintain a negative pressure state, and the glass bottle was placed on a stirrer and stirred for 0.1 hour. There was a large amount of bubbling, and the system transitions from two phases to a homogeneous phase.

Under normal temperature conditions, the glass bottle containing the absorbent from Example 14, after absorbing CO₂ as described in Application Example 1, was connected to a water pump. The glass bottle was vacuumed to maintain a negative pressure state, and the glass bottle was placed on a stirrer and stirred for 0.5 hours. There was a large amount of bubbling, and the system transitions from two phases to a homogeneous phase. The above indicated that, the absorbents produced in the present application can desorb most of carbon dioxide under normal temperature and negative pressure conditions, achieving the regeneration of carbon dioxide.

### Application Example 3

The absorbents obtained from Examples 1-33 and Comparative Examples 7-9 were placed in glass bottles under a carbon dioxide atmosphere and stirred for 3 hours at 20°C, and the changes in phase state were observed.

It can be observed that, Comparative Examples 7-9, which used carboxylic acid strong base salts as the absorbents, produced solids after absorbing carbon dioxide. The absorbents obtained from Examples 1-33 in the present application did not produce solids after absorbing carbon dioxide. This was due to the poor asymmetric structure and weak interaction between cations and anions in the absorbents produced in the present application, making it less likely for weak acid weak base salts of carboxylic acid to produce solid products after absorbing carbon dioxide.

### Application Example 4

50 grams of the absorbent obtained from Example 1 was injected into a glass bottle, and the glass bottle was connected to a carbon dioxide balloon to put the absorbent solution under the pressure of carbon dioxide. Stirring was performed for 3 hours at temperatures of 0°C, 10°C, 40°C, and 50°C, respectively, and the weights of the glass bottles were recorded before and after absorbing carbon dioxide. Based on the system's weight gain, the carbon dioxide absorption quantity was obtained. The results of the carbon dioxide absorption quantity of the absorbent obtained from Example 1 at different temperatures were shown in Table 4.

**Table 4: Results of the absorbent's carbon dioxide absorption at different temperatures**

| Absorption temperature | CO₂ absorption quantity /g | CO₂ absorption rate /% |
|---|---|---|
| 0 °C | 2.44 | 4.9 |
| 10 °C | 2.26 | 4.5 |
| 20 °C | 2.13 | 4.3 |
| 40 °C | 1.97 | 3.9 |
| 50 °C | 1.67 | 3.4 |

It can be seen that the absorbents produced in the present application can achieve good absorption effects of CO₂ within the temperature range of 0°C-50°C.

### Application Example 5

For absorption under carbon dioxide partial pressure equal to or lower than 0.1 MPa, 50 grams of the absorbent obtained from Example 1 was injected into a glass bottle, which was then connected to balloons containing different concentrations of carbon dioxide and nitrogen mixtures, respectively, and stirring was performed at 20°C for 3 hours. Based on the system's weight gain, the carbon dioxide absorption quantity was obtained. The concentrations of carbon dioxide in the balloons were measured.

For absorption under carbon dioxide partial pressure higher than 0.1 MPa, an acid gas partial pressure of 0.5 MPa was used as an example, at 20°C, 10 grams of the absorbent obtained from Example 1 were injected into a stainless steel pressure vessel with a valve, the magnon remains stationary, the air inside the vessel was evacuated using a water pump, the vessel was filled with 0.5 MPa of carbon dioxide, the valve was closed immediately and the piping was disconnected, the stainless steel pressure vessel was weighed and the weight was recorded as initial weight. Magnetic stirring was then activated to ensure adequate contact between the absorption liquid and carbon dioxide. The piping was reconnected, and the valve was opened to continuously introduce carbon dioxide, maintaining a pressure of carbon dioxide at 0.5 MPa in the system. After 3 hours, the valve was closed and the piping disconnected, and the stainless steel vessel was weighed and recorded. The difference in weight before and after the absorption process provided the carbon dioxide absorption quantity at 0.5 MPa.

The results of the carbon dioxide absorption quantity of the absorbent obtained from Example 1 at different partial pressures of carbon dioxide were shown in Table 5.

**Table 5: CO₂ absorption quantity of absorbent at different partial pressures**

| CO₂ partial pressure in CO₂ balloon | CO₂ absorption quantity /g | CO₂ absorption rate /% |
|---|---|---|
| 0.014 MPa | 0.75 | 1.5 |
| 0.036 MPa | 1.0 | 2.0 |
| 0.1 MPa | 2.13 | 4.3 |
| 0.5 MPa | 0.60 | 6.0 |
| 1.0MPa | 0.79 | 7.9 |

Based on Table 5, it can be seen that the absorbents produced in the present application can absorb carbon dioxide within the carbon dioxide partial pressure range of 0.01 MPa to 1.0 MPa, and has a better absorption quantity for higher concentration carbon dioxide.

### Application Example 6

10 grams of the absorbent obtained from Example 1 were injected into a glass bottle, connected to a balloon containing a certain concentration of sulfur dioxide and nitrogen mixture at 20°C, and stirred for 3 hours. The weights of the glass bottle before and after absorbing sulfur dioxide were recorded, and based on the system's weight gain, the sulfur dioxide (concentration of 3%) absorption quantity at a partial pressure of 0.003 MPa was obtained.

10 grams of the absorbent obtained from Example 1 were injected into a glass bottle and connected to a balloon containing hydrogen sulfide, stirred for 3 hours at 20°C, and the weights of the glass bottle before and after absorbing hydrogen sulfide were recorded, and the hydrogen sulfide absorption quantity was obtained based on the system's weight gain.

The acid gas absorption quantity of the absorbent obtained from Example 1 at 20°C was shown in Table 6.

**Table 6: The acid gas absorption quantity of the absorbent**

| Acid gas | Acid gas absorption quantity /g | Acid gas absorption rate /% |
|---|---|---|
| Sulfur dioxide | 1.17 | 11.7 |
| Hydrogen sulfide | 0.33 | 3.3 |

It can be seen from Table 6 that the absorbent produced in the present application can effectively absorb sulfur dioxide and hydrogen sulfide.

Obviously, the above examples are merely examples provided for clear illustration, and are not intended to limit the implementation methods. For those skilled in the art, other variations or modifications in different forms can be made based on the above description. It is neither necessary nor feasible to exhaust all implementation methods here. The obvious variations or modifications derived from this are still within the scope of protection of the present invention.

## Claims

1. A compound for capturing acid gases, wherein the compound has a structure shown in Formula (I), 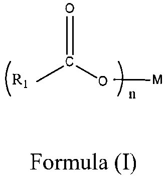 wherein, R₁ is an alkyl group with the number of carbon atoms greater than or equal to 5, M is at least one of protonated organic amine groups and protonated nitrogen-containing heterocyclic groups, and n is an integer from 1 to 4.

2. The compound for capturing acid gases according to claim 1, wherein R₁ is a branched alkyl group; and/or,
the organic amine is at least one of fatty amine and alcohol amine; and/or,
the nitrogen-containing heterocycle is at least one of 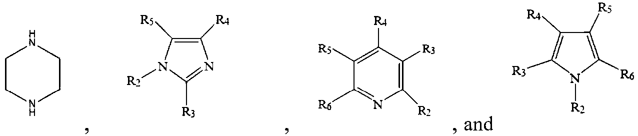
wherein R₂, R₃, R₄, R₅, R₆ are any one of alkyl, substituted alkyl, or hydrogen atom.

3. The compound for capturing acid gases according to claim 2, wherein the number of carbon atoms of R₁ is 6-20; and/or,
the organic amine is at least one of monoamine, diamine, and polyamine.

4. The compound for capturing acid gases according to claim 1, wherein the compound has any one of the following structures:

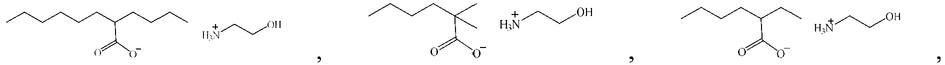

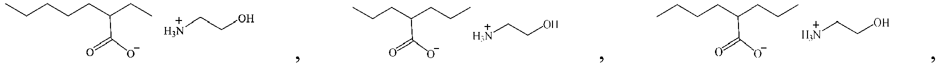

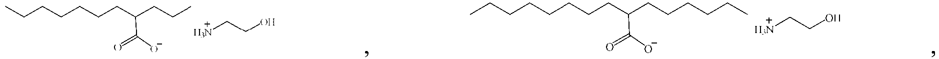

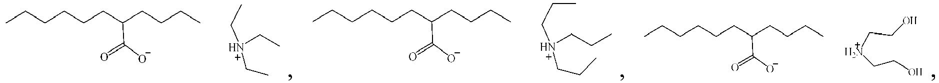

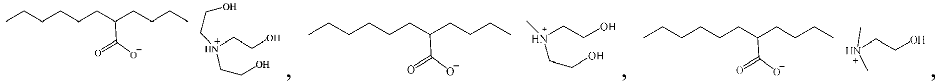

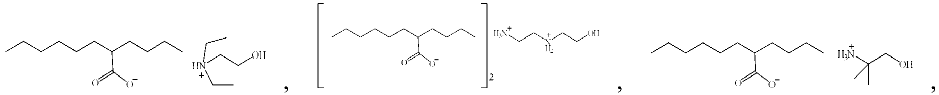

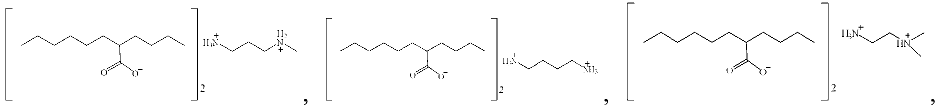

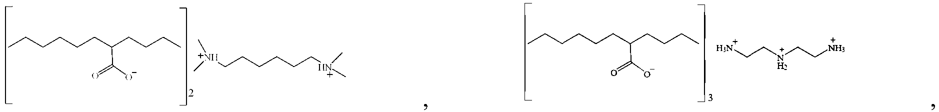

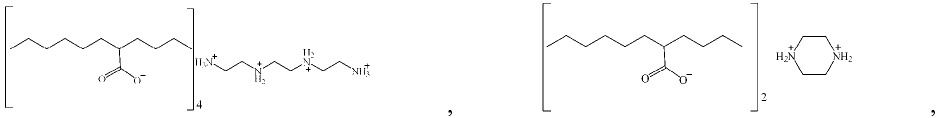

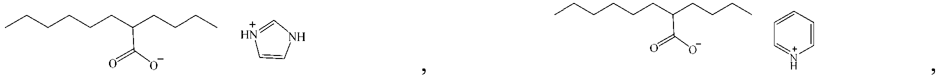

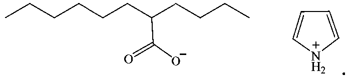

5. An absorbent for capturing acid gases, wherein the absorbent comprises the compound for capturing acid gases according to any one of claims 1 to 4.

6. A method for preparing the absorbent for capturing acid gases according to claim 5, wherein the method comprises the following steps:
mixing a carboxylic acid and an organic weak base to obtain the absorbent, where the carboxylic acid has a molecular formula of R₁COOH, and R₁ is an alkyl group with a number of carbon atoms greater than or equal to 5.

7. The method according to claim 6, wherein the absorbent also comprises water; and/or,
the carboxylic acid is a fatty acid; and/or,
the organic weak base is at least one of an organic amine compound and a nitrogen-containing heterocyclic compound.

8. The method according to claim 7, wherein the carboxylic acid is at least one of 2,2-dimethylhexanoic acid, 2-ethylhexanoic acid, 2-ethylheptanoic acid, 2-propylvaleric acid, 2-propylhexanoic acid, 2-propylnonanoic acid, 2-butyl-octanoic acid, and 2-hexyldecanoic acid; and/or,
the organic amine compound is at least one of triethylamine, tripropylamine, monoethanolamine, diethanolamine, triethanolamine, N-methyldiethanolamine, N,N-dimethylethanolamine, N,N-diethylethanolamine, hydroxyethyl ethylenediamine, 2-amino-2-methyl-1-propanol, N-methyl-1,3-propanediamine, 1,4-butanediamine, N,N,N',N'-tetramethylhexanediamine, diethylenetriamine, and triethylenetetramine; and/or,
the nitrogen-containing heterocyclic compound is at least one of a pyridine ring-containing compound, an imidazole ring-containing compound, a pyrrole ring-containing compound, and piperazine; and/or,
a molar ratio of the carboxylic acid, the organic weak base, and water is 1:(0.25-1):(0-20).

9. Use of the compound for capturing acid gases prepared according to any one of claims 1 to 4, the absorbent for capturing acid gases according to claim 5, and the method for preparing the absorbent for capturing acid gases according to any one of claims 6 to 8 in absorbing and regenerating acid gases, wherein the acid gases are at least one of carbon dioxide, sulfur dioxide, or hydrogen sulfide.

10. The use according to claim 9, wherein absorbing acid gases comprises steps of contacting the absorbent with the acid gases to be absorbed at an acid gas partial pressure of 0.003 MPa - 1.0 MPa and a temperature of 0-50°C and performing absorption; and/or,
regenerating acid gases comprises a step of desorbing the absorbent after absorbing the acid gases under stirring for 0.1-2 hours at 80-100 °C, or, regenerating acid gases comprises a step of desorbing the absorbent after absorbing the acid gases under stirring for 0.1-1 hour at 10-80 °C under vacuum conditions.
